# FASCICULE DE BREVET EUROPEEN

(11) **EP 1 255 437 B1**
(45) Date de publication et mention de la délivrance du brevet: **14.04.2010**
(21) Numéro de dépôt: 00964344.6
(22) Date de dépôt: 22.09.2000
(51) Int. Cl.: A01K 67/027, C07K 14/47, C12N 5/10, A61K 49/00, G01N 33/50

(54) **ANIMAL TRANSGENIQUE EXPRIMANT UNE FORME MULTI-MUTEE DE LA PRESENILINE 1**
TRANSGENES TIER WELCHES EIN MEHRFACH-MUTIERTES PRESENILIN-1 EXPRIMIERT
TRANSGENIC ANIMAL EXPRESSING A MULTIPLE MUTATED FORM OF PRESENILIN 1

(30) Priorité: 27.09.1999 FR 9912017; 09.02.2000 US 181306 P
(43) Date de publication de la demande: 13.11.2002
(73) Titulaire: Aventis Pharma S.A., 92160 Antony (FR)
(72) Inventeur: ECKERT, Anne, 68163 Mannheim (DE); MULLER, Walter, 67550 Worms-Herrnsheim (DE); CZECH, Christian, 79639 GRENZACH-WHYLEN (FR); PRADIER, Laurent, F-91370 Verriéres (FR); TREMP, Gunter, F-91120 Palaiseau (FR)
(74) Mandataire: Bouvet, Philippe
(86) Numéro de dépôt international: PCT/FR2000/002623
(87) Numéro de publication internationale: WO 2001/022811

(56) Documents cités:
- WO-A-98/17782
- WO-A-98/51781
- ECKERT A ET AL: "Enhanced vulnerability to cell death in lymphocytes from PS-1 mutant transgenic mice" SOCIETY FOR NEUROSCIENCE ABSTRACTS, vol. 25, no. 1-2, 1999, page 1846 XP000921143
- DALIE J.E. (PROGRAM MANAGER - SOCIETY FOR NEUROSCIENCE): "Publication dates for the 1999 Abstract Volumes" SOCIETY FOR NEUROSCIENCE, vol. 25, no. 1-2, 16 - 23 août 1999, XP002157614
- CZECH C ET AL: "Characterization of human presenilin 1 transgenic rats: Increased sensitivity to apoptosis in primary neuronal cultures." NEUROSCIENCE, vol. 87, no. 2, novembre 1998 (1998-11), pages 325-336, XP000914789
- LAMB B ET AL: "Amyloid production and deposition in mutant amyloid precursor protein and presenilin-1 yeast artificial chromosome transgenic mice" NAT. NEUROSCI., vol. 2, no. 8, août 1999 (1999-08), pages 695-697, XP002140369
- LEUTNER S ET AL: "Altered antioxidant enzyme activity and radical oxygen formation in PS-1 mutant transgenic mice" SOCIETY FOR NEUROSCIENCE ABSTARCTS, vol. 25, no. 1-2, 1999, page 1857 XP000921150
- ZAMAN S ET AL: "The effects of presenilin 1 mutations on synaptic physiology assessed in transgenic mouse models" SOCIETY FOR NEUROSCIENCE ABSTRACTS, vol. 24, no. 1-2, 1998, page 471,

## Description

La présente invention concerne le domaine des modèles animaux transgéniques et plus particulièrement, les modèles animaux de la maladie d'Alzheimer. L'invention se rapporte à un animal transgénique non-humain exprimant une forme de la préséniline 1 portant au moins cinq mutations parmi les mutations de la préséniline 1 identifiées dans les formes familiales de la maladie d'Alzheimer et présentant une susceptibilité accrue à l'apoptose. Le-dit animal permet de détecter un phénomène apoptotique dans un tissus périphérique renouvelable.

La maladie d'Azlheimer (AD) est une maladie neurodégénérative progressive qui affecte une large proportion de la population âgée. Cette maladie est caractérisée sur le plan clinique par une perte de la mémoire et un déclin des fonctions cognitives, sur le plan neuropathologique par la présence dans le cerveau de dépôts neurofibrillaires intracellulaires et de dépôts extracellulaires du peptide β-amyloïde (Aβ) formant les plaques amyloïdes (Yanker et al., 1996) ainsi qu'une perte neuronale prononcée. A ces signes s'ajoutent un nombre important d'autres changements anormaux incluant une altération des mécanismes de protection contre les radicaux libres..

Les plaques amyloïdes sont majoritairement composées des peptides Aβ à 40 ou 42 résidus qui sont générés lors du processus protéolytique de la protéine précurseur du peptide β-amyloïde (APP). Les dépôts extracellulaires de Aβ sont très spécifiques de l'AD et des désordres associés. Ils représentent la caractéristique précoce et invariable de toutes les formes de l'AD, incluant les formes familiales (FAD). Les FAD apparaissent de manière relativement précoce (entre 40 et 60 ans) et sont dues à des mutations dans le gène de l'APP dans 5 % des cas de FAD avec six mutations faux-sens simples ou doubles identifiées; dans le gène de la préséniline 1 (PS 1) dans 50 à 70 % des cas de FAD avec plus de 40 mutations différentes identifiées jusqu'à présent; et dans le gène de la préséniline 2 (PS 2) dans des cas plus rares de FAD avec 2 mutations faux-sens décrites (pour revue voir Price et Sisodia, 1998). Des mutations dans ces trois gènes ont été démontrées comme induisant des changements dans la protéolyse de l'APP, qui conduisent à une surproduction de Aβ, spécialement de la forme longue Aβ42, et à l'apparition précoce de la pathologie et des symptômes similaires à ceux des formes sporadiques de l'AD.

Dans les modèles animaux transgéniques décrits jusqu'à présent la symptomatologie de perte neuronale comparable à l'AD est exprimée uniquement au niveau des neurones ou dans leur entourage direct et en particulier le phénomène d'apoptose (Chiu et al., 1999). Cependant, ces modèles présentent plusieurs inconvénients dont notamment la nécessité d'élever un très grand nombre d'animaux le plus souvent sur des périodes de temps longues, pouvant aller jusqu'à 24 mois, afin de suivre la mise en place des symptômes de l'AD, le sacrifice systématique des animaux pour l'étude de la pathologie et ainsi des protocoles expérimentaux particulièrement fastidieux et coûteux.

La demande de brevet WO98/51781 décrit des animaux dont la PS1 ne porte qu'une seule mutation (voir exemple 8).

Une autre demande de brevet - WO98/17782- décrit une souris exprimant une PS1 ne portant qu'une seule mutation M146L et exprimant une APP portant la mutation Swedish.

L'abrégé publié par ZAMAN et al (SOCIETY FOR NEUROSCIENCE ABSTRACTS, vol. 24, no. 1-2, 1998, page 471) rapporte la création de souris sur-exprimant une PS1 portant la mutation A246E ou une délétion de l'exon 9.

Les abrégés publiés aux noms d'ECKERT et al (SOCIETY FOR NEUROSCIENCE ABSTRACTS, vol. 25, no. 1-2, 1999, page 1846) et de LEUTNER S ET AL (SOCIETY FOR NEUROSCIENCE ABSTRACTS, vol. 25, no. 1-2, 1999, page 1857) n'identifient pas les mutations dans PS1 et il n'est donc pas possible d'en déduire le nombre de mutations.

L'article de CZECH et al (NEUROSCIENCE, vol. 87, no. 2, novembre 1998 (1998-11), pages 325-336) décrit des rats transgèniques. Mais ces rats portent la PS non mutée. En effet il apparaît clairement à la lecture de ce document, en particulier page 327, que la protéine exprimée est la PS1 sauvage et non un mutant de cette protéine.

L'article de LAMB et al (NAT. NEUROSCI., vol. 2, no. 8, août 1999 (1999-08), pages 695-697) décrit des animaux double mutant H163R et M146L.

La présente invention résulte donc de la recherche d'un nouveau modèle animal représentatif de la neuropathologie permettant de mesurer les symptômes associés à l'AD et en particulier apoptose, dans des tissus périphériques.

Un premier objet de l'invention concerne donc un animal transgénique non-humain exprimant une forme de la préséniline 1 portant au moins cinq mutations parmi les mutations de la préséniline 1 identifiées dans les formes familiales de la maladie d'Alzheimer et présentant une susceptibilité accrue à l'apoptose.

Le-dit animal peut servir de modèle animal transgénique de la maladie d'alzheimer.

On entend par animal transgénique tout animal non-humain présentant une modification de son génome. La modification du génome peut résulter d'une altération ou une modification de un ou plusieurs gènes par "knock-in" ou par "knock-out". Cette modification peut être due à l'action d'agents altérants ou mutagènes classiques ou bien effectuée par mutagenèse dirigée, comme cela est décrit dans Matériels et Méthodes.

La modification du génome peut également résulter d'une insertion de gène(s) ou de remplacement de gène(s) dans sa (leur) forme sauvage ou mutée.

Les modifications du génome sont avantageusement effectuées sur des cellules souches reproductrices et préférentiellement sur les pronucléi.

Dans le cadre de la présente invention, le modèle animal est avantageusement un mammifère non-humain. En particulier il peut s'agir d'une souris, d'un rat ou d'un lapin obtenu selon les techniques classiques de transgénèse. A titre d'exemple illustrant l'un des procédés de transgénèse, on peut citer la méthode de microinjection d'une cassette d'expression comprenant les gènes modifiés dans les deux pronucléi fécondés, tel que cela est décrit dans Matériels et Méthodes.

A cet égard, le modèle animal de l'invention est obtenu par injection d'une cassette d'expression comprenant un acide nucléique. De manière préférentielle, cet acide nucléique est un ADN qui peut être un ADN génomique (ADNg) ou un ADN complémentaire (ADNc).

Dans le cadre du modèle de l'invention, l'ADN code pour tout gène de la PS1 de sorte que les cellules du modèle animal expriment la protéine multi-mutée, portant au moins cinq mutations parmi les mutations de la PS1 identifiées dans les formes familiales de la maladie d'alzheimer. La séquence de la protéine PS1 humaine non-mutée a été décrite par Sherrington et al en 1995. On entend par protéine multi-mutée, la protéine PS1 comprenant au moins cinq mutations combinées ou associées entre elles, c'est-à-dire présentent en même temps dans la dite protéine. Selon un mode préféré de l'invention, l'ADN code pour le gène de la PS1 qui comporte 5 mutations (PS1M5).

Les mutations dans le gène de PS1 peuvent être l'une des 40 mutations décrites jusqu'à présent dans la littérature. De manière préférentielle, les mutations dans le gène de PS1 sont M146L, H163R, A246E, L286V, C410Y, I143T, L235P, P264L, P267S, E317G, G384A, L392V, A426P et/ou P436S. Elles sont en combinaison partielle les unes aux autres.

Pour la réalisation d'un modèle selon l'invention, les mutations M146L, H163R, A246E, L286V, C410Y, combinées entre elles, sont préférées.

Dans le cadre du modèle de l'invention, l'ADN est placé sous le contrôle de séquences permettant son expression et en particulier de séquences promotrices de la transcription.

A titre de séquences promotrices, on peut citer tout particulièrement le promoteur HMG (Gautier et al., 1989), ainsi que le promoteur PDGF (Sasahara et al., 1991), le promoteur Thy-1 (Lüthi et al., 199) et le promoteur du gène du Prion (Scott et al., 1992).

Selon une mise en oeuvre particulièrement intéressante de l'invention, le modèle animal comprend le gène de la PS1 ayant les mutations M146L, H163R, A246E, L286V, C410Y placé sous le contrôle du promoteur HMG.

Le modèle animal selon l'invention est très avantageux car il correspond à un modèle pratique et représentatif des phénomènes de mort cellulaire de l'AD. En effet, ce modèle présente des symptômes associés à l'AD dont notamment l'apoptose des cellules et le stress oxydatif et permet en plus de mesurer ces symptômes dans les cellules des tissus périphériques renouvelables. Il est à noter que le stress oxydatif se manifeste également dans le cerveau de ces animaux. On doit entendre par tissus périphériques renouvelables, tout tissus présentant un renouvellement de ces cellules au cours du temps. A titre d'exemple de tissus périphérique renouvelable on peut citer la rate, le foie, le sang...... De manière préférée, le phénomène apoptotique est mesuré dans les cellules du sang et encore plus préférentiellement dans les lymphocytes. Parmi les lymphocytes, les lymphocytes T sont préférés pour l'invention.

Ainsi, les résultats décrits dans les exemples démontrent que la souris transgénique exprimant la PS1 multi-mutée, développe des altérations cellulaires retrouvées dans la maladie d'Alzheimer et, en particulier, présente une sensibilité accrue à l'apoptose. Ce phénotype n'est d'ailleurs pas observé avec un mutant pathologique naturel simple du genre M 146L. Ce phénotype est obtenu spécifiquement avec une forme non naturelle regroupant plusieurs mutations, et de préférence 5 mutations, individuelles sur le même ADNc. En outre, par l'expression ectopique du transgène grâce au promoteur ubiquitaire, ce modèle permet de détecter un phénomène apoptotique (lié à des mutations de la maladie d'Alzheimer) dans un tissu périphérique renouvelable. Ce modèle fournit donc une source beaucoup plus pratique de matériel (n'exigeant pas le sacrifice de l'animal) permettant donc un suivi longitudinal.

De plus, les altérations du métabolisme du Calcium et des radicaux libres observées dans ce modèle de manière très nette sont similaires à l'augmentation de la latence de la réponse calcique et du stress oxidatif observées chez les patients Alzheimer (Eckert et al., 1997 and 1998) ce qui renforce la pertinence de ce modèle.

La présente invention est donc également relative à l'utilisation du modèle animal, tel que décrit précédemment, pour la mise en évidence de composés destinés au traitement des maladies neurodégénératives, de préférence la maladie d'Alzheimer.

En effet, par ses propriétés avantageuses ce modèle permet, en comparaison des modèles connus, la mise en évidence de composés particulièrement adaptés au traitement de l'AD, notamment, telle que décrite chez l'homme.

Ces composés peuvent être des molécules chimiques, des molécules peptidiques ou protéiques, des anticorps, des molécules chimériques ainsi que des ADNs antisens ou des ribozymes.

La mise en évidence des composés décrits précédemment repose sur la mise en contact, notamment par une administration telle que par exemple une injection, du modèle animal de l'invention avec un composé ou un mélange de composés supposé(s) avoir une action et de mesurer ensuite le ou les effet(s) des composés notamment au niveau des tissus périphériques du modèle sur les différents changements biochimiques et/ou histologiques comme par exemple ceux décrits dans les parties Méthodes et Résultats dont l'apoptose, le taux de calcium intracellulaire, le taux de radicaux libres....

Un autre objet de l'invention concerne une cellule extraite du modèle animal tel que décrit précédemment, la-dite cellule exprimant une forme de la préséniline 1 portant au moins cinq mutations parmi les mutations de la préséniline 1 identifiées dans les formes familiales de la maladie d'Alzheimer et présentant une susceptibilité accrue à l'apoptose, ainsi que son utilisation pour la mise en évidence de composés destinés au traitement des maladies neurodégénératives, de préférence la maladie d'Alzheimer.

La mise en évidence de composés décrits précédemment repose sur la mise en contact des-dites cellules extraites du modèle animal de l'invention avec un composé ou un mélange de composés supposé(s) avoir une action et de mesurer ensuite le ou les effet(s) des composés au niveau des cellules entières, dans des homogènâts de cellules ou sur une fraction subcellulaire, sur différents paramètres tels que la mort cellulaire, la production du peptide Aβ, production de radicaux libres, etc...

Les résultats décrits dans les exemples démontrent les avantages du modèle de l'invention et supportent clairement l'utilisation de ce modèle transgénique comme outil de mesure et de suivi simple et rapide dans le cadre de stratégies thérapeutiques telles que notamment la mise au point d'agents anti-apoptotiques ou d'agents limitant la mort cellulaire liée à l'AD de façon plus générale.

La présente invention sera décrite plus en détail à l'aide des exemples qui suivent mais qui doivent être considérés comme illustratifs et non limitatifs.

### LEGENDE DES FIGURES

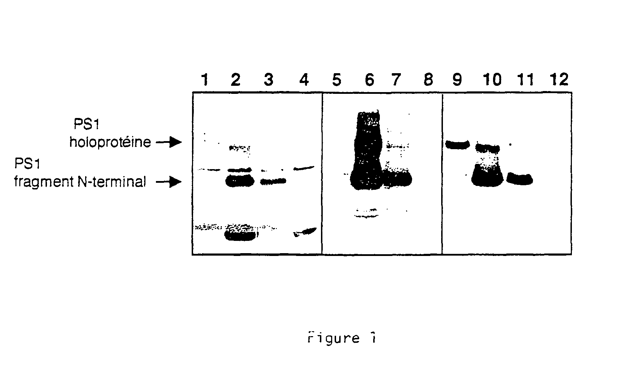

**Figure 1** **:** Analyse de l'expression de PS1 humaine chez les souris transgéniques PS1M5 (pistes 1,5,9), PS1M146L (pistes 2,6,10), PS1wt (pistes 3,7,11) et non transgéniques (pistes 4,8,12). Les tissus des souris transgéniques : lymphocytes (pistes 1-4), rate (pistes 5-8) et cerveau (pistes 9-12), ont été lysés et analysés par immunoblot en utilisant un anticorps spécifique pour la séquence humaine de PS1 (épitope dans la région N-term de PS1). L'holoprotéine PS1(approx. 50kDa) ainsi que le fragment N-terminal sont observables. L'expression du transgène est observable pour les trois transgéniques. Il y a absence de clivage endoprotéolytique pour la protéine PS 1 M5.

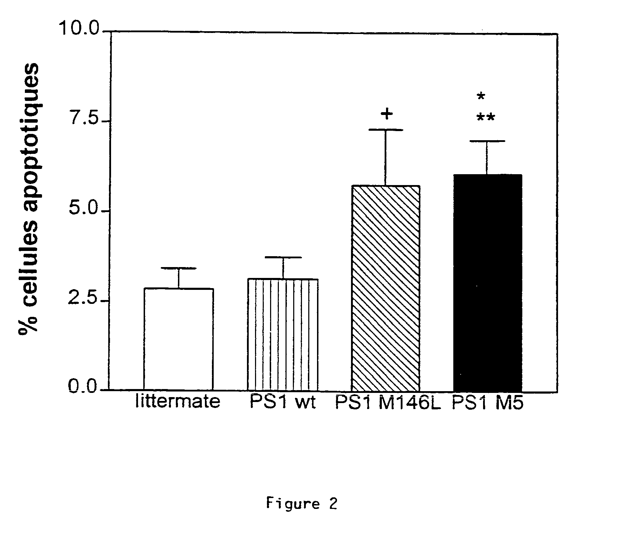

**Figure 2** **:** Apoptose accrue des lymphocytes issus de souris transgèniques PS1M146L et PS1M5 en conditions basales. Les taux d'apoptose en condition basale ont été mesurés dans des lymphocytes dissociés. Les lymphocytes PS1M5 montrent un taux d'apoptose plus élevé par rapport à PS1wt (*, p< 0.05) ou aux témoins littermates , non transgéniques, (**, p< 0.05)). Il en est de même pour les lymphocytes PS1M146L par rapport aux contrôles littermates (+, p< 0.05)

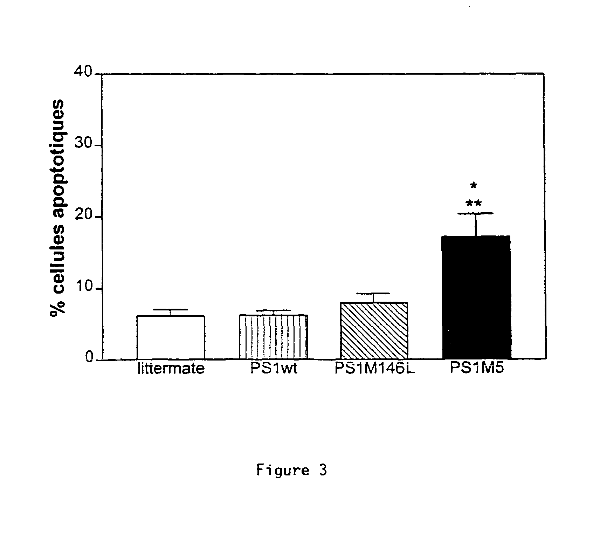

**Figure 3** **:** Apoptose après 2.5h d'incubation de lymphocytes issus de souris transgéniques. Après 2.5h d'incubation, les lymphocytes PS1M5 montrent un taux d'apoptose significativement plus élevé (***, p< 0.001) par rapport à PS1wt , PS 1 M 146L ou aux contrôles non transgéniques (littermates)

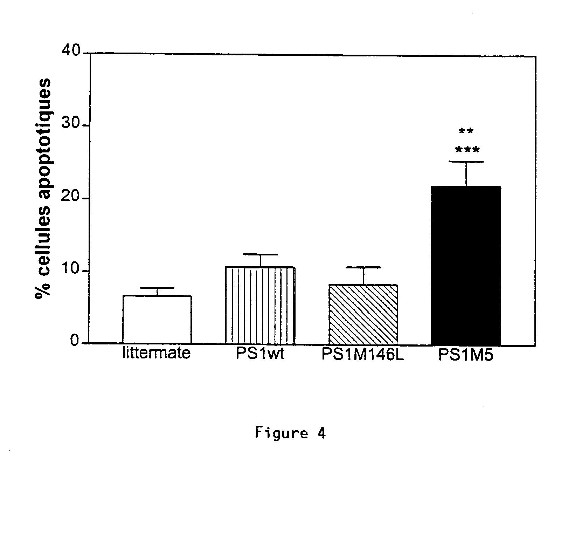

**Figure 4** **:** Apoptose induite par traitement au déoxy-ribose de lymphocytes issus de souris transgéniques. Après induction au deoxy-D-ribose (10mM), les taux d'apoptose sont significativement plus élevés dans le groupe PS1M5 que dans les autres groupes (**,p< 0.01 vs PS1 wt et PS1M146L ; ***, p<0.001 vs littermates).

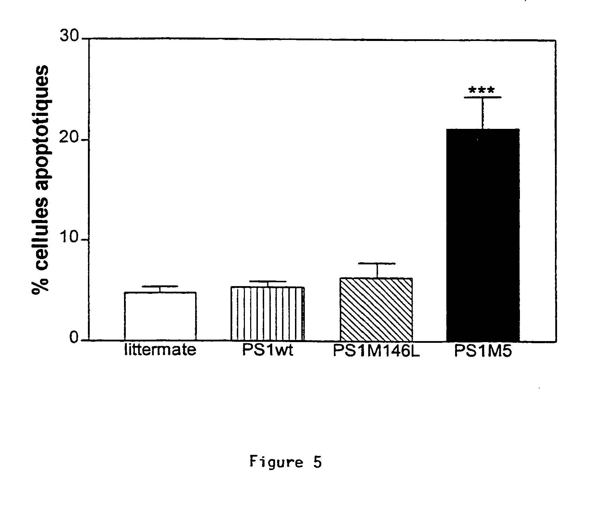

**Figure 5** **:** Apoptose induite par traitement au peroxide d'hydrogène de lymphocytes issus de souris transgéniques. Après induction au peroxide d'hydrogène (1mM), les taux d'apoptose sont significativement plus élevés dans le groupe PS1M5 que dans les autres groupes (***, p< 0.001). Il n'y a aucune différence entre les autres groupes..

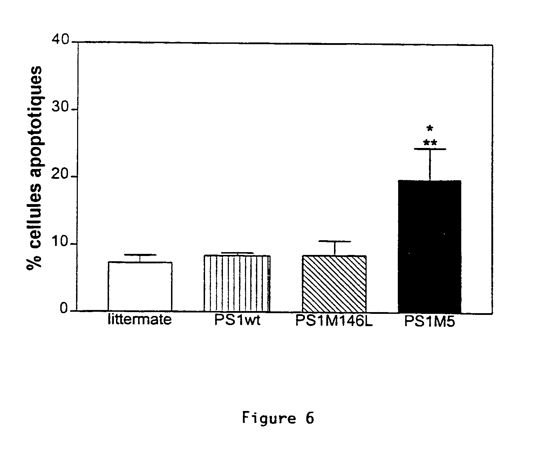

**Figure 6** **:** Apoptose induite par traitement à la dexaméthasone de lymphocytes issus de souris transgéniques. Après induction à la déxaméthasone (10⁻⁷ M), les taux d'apoptose sont significativement plus élevés dans le groupe PS1M5 que dans les autres groupes (*, p<0.05 vs PS1M146L et littermates ; **,p< 0.01 vs PS1wt). Il n'y a aucune différence entre les autres groupes.

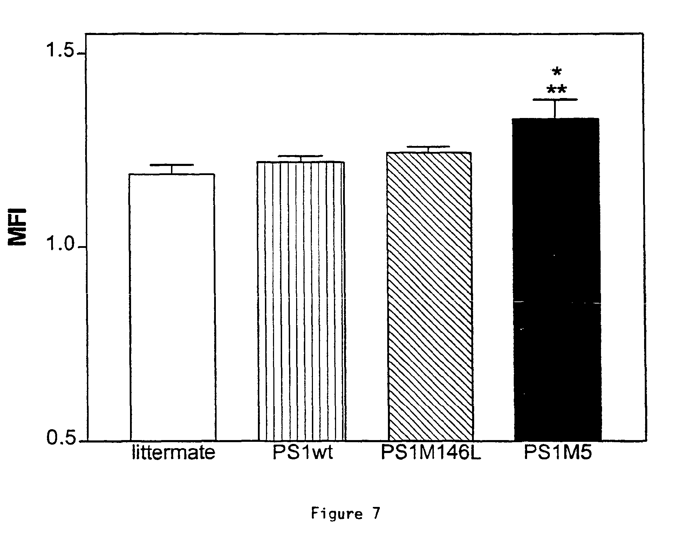

**Figure 7** **:** Augmentation des niveaux de radicaux libres dans les lymphocytes de souris transgéniques PS1M5. Les niveaux des espèces radicalaires oxygénées (Reactive Oxygen Species) ont été mesurés dans les lymphocytes de souris par cytométrie de flux (Rhodamine123) et exprimés en intensité moyenne de fluorescence (MFI). Les taux de ROS sont significativement plus élevés dans les lymphocytes de souris transgéniques PS1M5par rapport aux autres groupes (*, p<0.05 vs PS1wt; **,p<0.01 vs littermates).

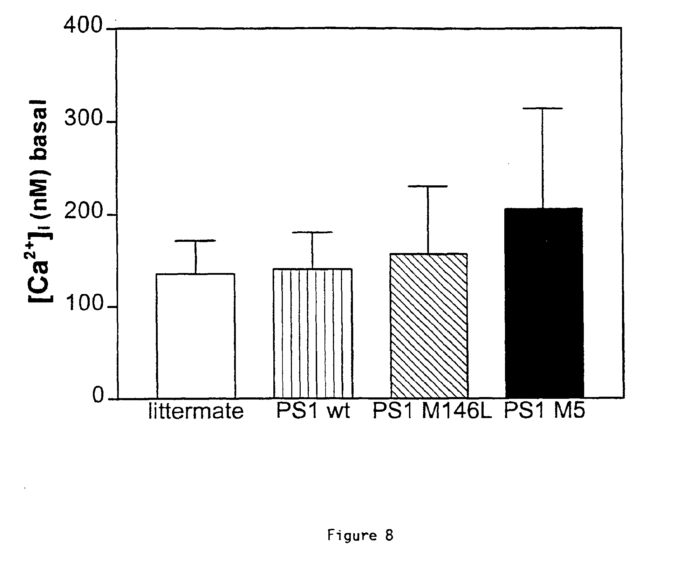

**Figure 8** **:** Mobilisation accrue de calcium intracellulaire dans les lymphocytes de souris transgéniques PS1M5. Les niveaux de [Ca²⁺]ᵢ ont été déterminés en conditions de repos (basal) Les niveaux de calcium intracellulaire sont plus élevés dans les lymphocytes de souris transgéniques PS1M5 que dans les autres groupes. Il n'y a aucune différence entre les autres groupes.

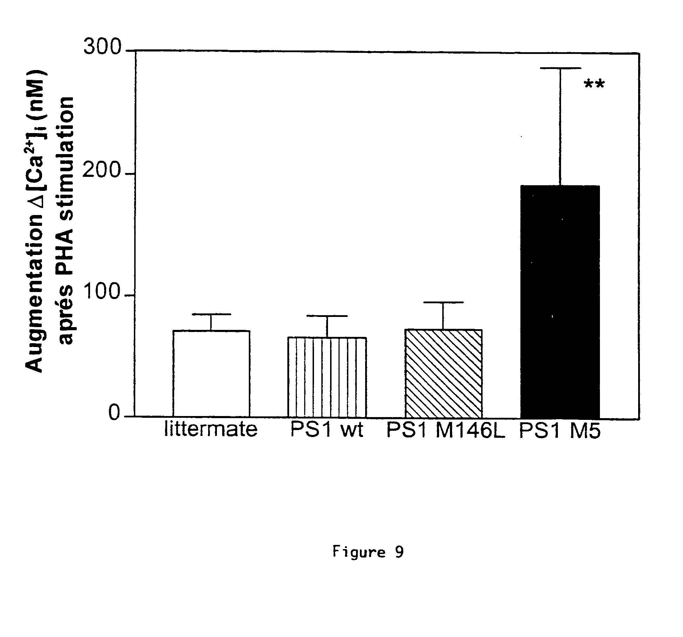

**Figure 9** **:** Réponse calcium intracellulaire accrue après stimulation au PHA dans les lymphocytes de souris transgéniques PS15. La différence entre niveaux de [Ca²⁺]ᵢ après et avant stimulation mitogénique au PHA (15microg/ml) a été représentée. Les lymphocytes de souris transgéniques PS1M5 répondent plus fortement au stimulus que les autres groupes (p< 0.01). Il n'y a aucune différence entre les autres groupes.

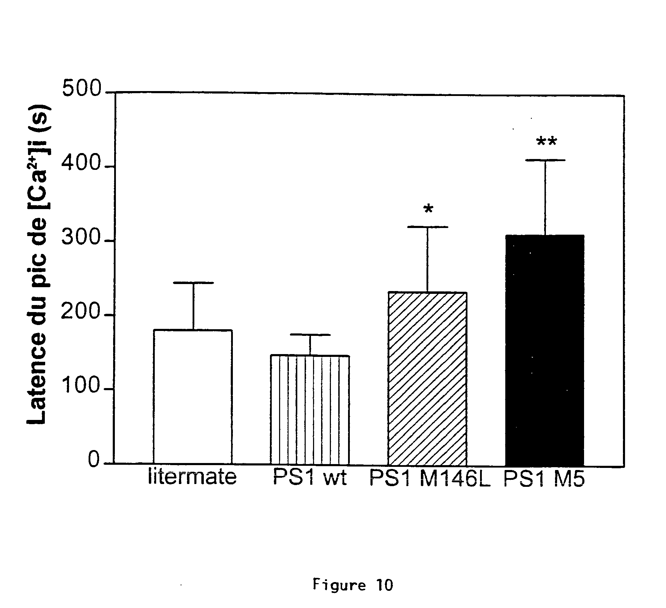

**Figure 10** **:** Latence de la mobilisation de calcium intracellulaire plus lente dans les lymphocytes de souris transgéniques PS1M5 et PS1M146L. L'intervalle de temps pour atteindre le pic de[Ca²⁺]ᵢ après stimulation mitogénique au PHA est plus élevé dans les lymphocytes de souris transgéniques PS1M5 (**, p< 0.05 vs PS1wt et littermates) ainsi qu'à un moindre degré pour les souris transgéniques PS1M146L (*, p<0.05 vs PS1wt). Il n'y a aucune différence entre souris PS1wt et contrôles non transgéniques (littermates).

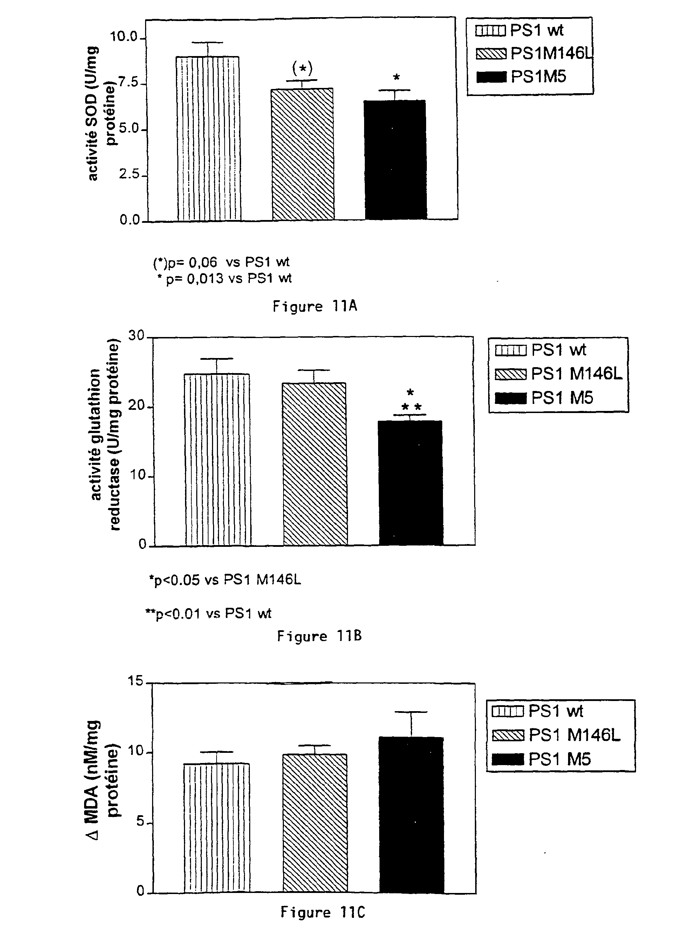

**Figure 11** **:** Effet des mutations humaines de PS1 dans le cerveau des souris transgéniques sur les mécanismes de protection des radicaux libres. A) Niveaux d'activité de l'enzyme super oxyde dismutase, SOD. B) Niveaux d'activité de l'enzyme glutathion réductase. C)Niveaux de peroxydation lipidique après stimulation au FeCl₃. Une baisse significative des mécanismes de détoxification des radicaux libres (SOD et GR) est observée dans le cerveau des souris transgéniques PS1M5 (*, p< 0.05 vs PS1wt; **, p<0.05 vs PS1M146L) avec une tendance à la diminution dans les souris PS1M146L. Réciproquement, les niveaux de peroxydation lipidique stimulée (due à la présence de radicaux libres) est augmentée chez les souris PS1M5.

### MATERIEL ET METHODES

### 1. Mutagenèse de Préséniline1-PS1

L'ADNc de PS1 humain contenant un consensus Kozak au niveau de l'ATG initial, a été précédemment décrit (Pradier et col. 1999). La mutagenèse de PS 1 a été effectuée en utilisant le kit de mutagenèse in vitro Sculptor™ (Amersham, France). La région codante de PS1 a été sous-clonée dans le vecteur Bluescript (Stratagène) et un ADN simple brin préparé. Les 5 mutations utilisées dans le cadre de l'invention, ont été introduites à l'aide d'oligonucléotides contenant les mutations souhaitées suivant les instructions du fournisseur :
M146L, ^{5'} GAGGATAGTCG*TGACAACAAT ^{3'} ; SEQ ID N°1
H163R, ^{5'} AAGCCAGGCC*TGGATGACCTT ^{3'} ; SEQ ID N°2
A246E, ^{5'} GATGAGCCAT*GCAGTCCATTG ^{3'}; SEQ ID N°3
L286V, ^{5'} GGAGTAAATGC*GAGCTGGAAA ^{3'}; SEQ ID N°4
C410Y, ^{5'} GGCTACGAAT*CAGGCTATGGT ^{3'}, SEQ ID N°5
le * dénote la position de la mutation nucléotidique introduite sur le brin complémentaire. Pour obtenir la construction contenant les cinq mutations combinées (PS1M5 pour multimutant5), cinq mutagenèses successives ont été effectuées. L'ensemble de la séquence de l'ADNc de PS1 a été vérifiée à chaque mutagenèse pour garantir l'absence de mutations non souhaitées.

### 2. Génération et identification des souris transgéniques

Pour la construction des transgènes, lesADNc de PS1 wild type, PS1M146L et PS1M5 ont été sousclonés dans les sites de restriction SmaI/BamHI du multisite de clonage du vecteur d'expression transgénique HMG (Czech et col. , 1997). Les ADNc sont sous le contrôle du promoteur partiel HMG-CoA reductase qui permet une expression ubiquitaire du transgène. Pour la microinjection, la cassette d'expression a été purifiée par électrophorèse sur gel après restriction avec l'enzyme NotI pour éliminer les séquences non importantes du vecteur. Le transgène purifié a été repris à la concentration finale de 2.5 ng/microl en 10 mM Tris-HCl (pH 7.4), 0.1 mM EDTA et injecté dans l'un des deux pronucléi d'ovocytes fécondés de souris. Les embryons survivants sont transplantés dans l'oviducte d'une mère adoptive. La présence du transgène a été analysée par PCR et Southern. La PCR a été effectuée en utilisant des oligonucléotides spécifiques de la séquence de PS1 humain présentant les séquences SEQ ID N°6 ^{5'}-TAA TTG GTC CAT AAA AGG C-^{3'} et SEQ ID N°7 ^{5'} - GCA CAG AAA GGG AGT CAC AAG-³' générant un fragment d'amplification de 550 pb. Pour l'analyse de Southern, un fragment PstI-SalI de 1.2 kb, correspondant au premier intron de la cassette d'expression HMG a été marqué au alpha-P³² et utilisé comme sonde pour la détection du transgène et du gène HMG-CoA reductase endogène. Grâce à cette dernière analyse, l'absence de tout réarrangement majeur ou délétion au sein du transgène peut être garantie. Les souris ont été élevées conformément aux règles françaises d'entretien des animaux.

### 3. Immunoblot

Le tissu cérébral et la rate de souris transgéniques (PS1 wt, PS1M146L et PS1M5) et de souris contrôles non transgéniques (littermate) a été homogénéisés sur de la glace dans une solution de sucrose 0.32 M contenant des inhibiteurs de protéase (Complete^{™}, Boehringer-Mannheim, Allemagne). Les débris cellulaires ont été éliminés par centrifugation à 4°C pendant 5 min à 1500g. Les lysats de lymphocytes ont été préparés de la même façon à partir de la fraction de cellules purifiées. La concentration en protéine dans le surnageant a été mesuré à l'aide du test de protéine BCA (Pierce, USA). Pour la détection de PS1, 25 µg d'extrait protéique ont été incubés à 56°C pendant 20 min dans du tampon de dépôt Laemmli contenant de l'urée 8M et du dithiothreitol 50 mM. Les protéines ont été fractionnées par électrophorèse sur gel polyacrylamide (SDS-PAGE). Après transfert des protéines sur filtre de nitrocellose (Amersham, France), le filtre a été chauffé dans du PBS pendant 5 min afin d'augmenter la sensibilité et immédiatement saturé avec 5% (w/V) de lait écrémé en poudre dans du TBST 50mM Tris-HCl pH 8.1, 150 mM NaCl, 0.05% (V/V) Tween 20 pendant lh et incubé la nuit à 4°C avec l'anticorps (Ac) primaire anti-PSI humain, MAB1563 (Chemicon, USA), dilué au 1/10000 en tampon TBST seul. La liaison de l'Ac a été détecté avec un Ac-anti IgG conjugué à la peroxidase de Raifort (Amersham, France) suivi d'un système de détection par chemiluminescence (Amersham, France) selon les instructions du fabricant.

### 4. Préparation des lymphocytes

Les lymphocytes ont été préparés à partir de rate de souris fraîchement dissociée. La rate est homogénéisée en tampon RPMI puis l'homogénat est passé à travers un filtre 10 microns. L'homogénat cellulaire est lavé plusieurs fois par centrifugation et resuspension. Après lyse des erythrocytes présents, le nombre de cellules est déterminé par comptage au microscope.

Pour obtenir les lymphocytes T, les cellules B ont été contre-sélectionnées par fixation sur des billes magnétiques portant des anticorps anti-cellules B (Dynabeads Mouse pan B, Dynal, Norvège) et séparation des billes. Les cellules restantes sont à plus de 80% des lymphocytes T (CD3 positives) comme déterminé par analyse par cytométrie de flux.

### 5. Mesure de l'apoptose

Pour déterminer le contenu en ADN en phase G1, qui définit le pourcentage de cellules apoptotiques, après traitements aux différents temps, les lymphocytes T sont séparés par centrifugation et les culots cellulaires repris en tampon de lyse ( 0.1 % citrate de sodium, 0.1% Triton X-100) contenant 50 µg/ml de iodure de propidium (Sigma, Munich). Les échantillons sont conservés à 4°C pour 1-2 heures avant analyse par cytométrie de flux (FACSCalibur, Benckton Dickinson, logiciel Cell Quest). La mort cellulaire a été induite par différents traitements : 2-deoxy-D-ribose (d-Rib, 10mM), peroxide d'hydrogène (H₂O₂, 1 mM) et dexamethasone (dex, 10⁻⁷ M) pendant 2.5h. La mort cellulaire déterminée en absence de traitement a été définie comme apoptose spontannée *in vitro.*

### 6. Mesure de calcium intracellulaire

La fraction de cellules T est reprise en tampon RPMI et incubée en présence de Fura-2-AM (Molecular Probe, Leiden, Pays-Bas). L'incubation est terminée par ajout de tampon HBSS et lavage de la suspension par centrifugation plusieurs fois pour éliminer le colorant du milieu. La fraction de cellules T est finalement reprise en tampon HBSS et conservée sur glace jusqu'à la mesure de calcium intracellulaire.

La fluorescence Fura-2 a été mesurée comme précédemment décrit (Eckert et al., 1993) en utilisant un spectromètre à luminescence SLM-Aminco avec longueurs d'onde d'excitation à 340nm et 380 nm et d'émission à 510nm. La concentration intracellulaire de calcium [Ca²⁺]ᵢ a été calculée à partir de la méthode des ratios de Grynkiewicz comme décrit précedemment (Eckert et cl.1997) en utilisant une valeur de Kd de 224 nM. Comme stimulateur de mobilisation de calcium dans les lymphocytes, la PHA-P (Sigma, Munich) a été rajoutée à la concentration de 15 microgram/ml.

### 7. Mesure des radicaux libres

La production de radicaux libres (ROS) a été quantifiée par cytométrie de flux (FACSCalibur, Benckton Dickinson ) en utilisant comme révélateur fluorescent la dihydrorhodamine 123 (DHR, Molecular Probes). Les cellules sont resuspendues dans 1 ml de tampon HBSS en présence de DHR (concentration finale 10microM) et incubées à 37°C pour 15min en bain agitant. La conversion de DHR en son dérivé fluorescent rhodamine 123 est alors quantifiée et exprimé en intensité moyenne de fluorescence (MFI).

### 8. Preparation du tissu cérébral.

Les souris ont été utilisées à l'age de 3-4 mois et sacrifiées par décapitation. Les cerveaux ont été prélevés et lavés extensivement dans du tampon sur glace. Le tissu (cervelet inclus) a été pesé et immédiatement congelé à -20°C. Le tissu a été homogénéisé à l'aide d'un homogénéisateur Potter-Elvehjem dans du tampon Tris-HCl à 5 et 20 mM, respectivement, pour obtenir des homogénéat dilué (poids/volume) au 1/l0^{e} et 1/5^{e} respectivement.

### 9. Test d'activité CuZn SOD et glutathion reductase

Les homogénats de cerveau (1/5 p/v) ont été centrifugés à 8.500xg pendant 10minutes à 4°C. Le surnageant a été utilisé pour mesurer les activités SOD et glutathion reductase (GS). L'activité SOD a été mesurée à l'aide d'un kit du type SOD-525 (Calbiochem, Allemagne). Ce kit utilise un réactif spécifique (R1) qui subit une auto-oxidation alkaline qui est accélérée par la superoxide dismutase. Un deuxième réactif (R2) est utilisé pour éliminer les interférences causées par les mercaptans, comme le glutathion. Une unité d'activité SOD-525 est définie comme l'activité doublant le taux d'auto-oxidation de R1. L'activité GR a été similairement dosée à l'aide d'un kit spécifique (Calbiochem, Allemagne) . Ce kit mesure le taux d'oxidation de NADPH en NADP+ qui est accompagné d'une diminution d'absorbance à 340 nm qui est détectée par spectrophotométrie. Une unité d'activité GR est définit par la réduction d'une micromole de GSSG à 25°C, pH7.6.

### 10. Mesure de la peroxidation lipidique (LPO) basale et stimulée dans les tissus cérébraux

Les homogénats de cerveau (1/10, p/v) sont incubés en tampon contenant (LPO stimulée) ou pas (LPO basale) FeCl₃ et 100 µM pendant 30 min à 37°C dans un bain aqueux agité. Après incubation, les homogénats sont centrifugés à 3000xg pendant 10 min. Les surnageants sont utilisés pour détecter la peroxidation lipidique par mesure de la concentration de malondialdéhyde (MDA) à l'aide d'un kit de type LPO-586 (Calbiochem). Ce kit utilise un chromophore spécifique qui réagit avec le MDA à une temprétaure modérée (45 °C)

### EXEMPLES

### Exemple 1 : immunodétection des protéines correspondant aux transgènes PS1wt, PS1M146L et PS1M5

Des souris transgéniques PS1wt, PS1M146L et PS1M5 (multimutant) ont été générées. Le transgène est sous le contrôle du promoteur humain HMG-CoA reductase, un gène de maintenance qui confèrre une forte expression ubiquitaire incluant le cerveau (Gautier et al., 1989, Czech et col. 1997 et 1998). Une analyse des niveaux d'expression des transgènes (PS1 humaine) a été effectuée sur les tissus de rate et de cerveau ainsi que sur la fraction de cellules lymphocytaires à l'aide d'un anticorps spécifique de la séquence de PS1 humaine ne reconnaissant pas l'homologue de souris (Fig1) Dans le cerveau (Figl, pistes 9-12), le fragment N-terminal de PS1 est l'espèce prépondérante avec des niveaux plus élevés pour la PS1M146L comparée à PS1wt. Pour PS1M146L, l'holoprotéine (protéine complète, approx. 50 kDa) est détectable démontrant une saturation du processus d'endoprotéolyse à forte expression de PS1 comme précédemment décrit. Par contre, pour PS1M5, le multimutant, il y a une absence totale d'endoprotéolyse et seule l'holoprotéine est détectable. Des résultats similaires ont été observés dans la rate et les lymphocytes avec des niveaux d'expression variables mais détectables dans tous ces tissus.

### Exemple 2 : Apoptose spontanée accrue dans les lymphocytes isolés de souris transgéniques PS1 multi-mutées.

Cet exemple a pour but de démontrer que les cellules périphériques du modèle animal exprimant la PS1 multi-mutée présentent une apoptose spontanée acrue et persistante dans le temps.

Les lymphocytes isolés de souris transgéniques exprimant PS1 wild type (sans mutation) ont le même niveau d'apoptose basale (2.8%) que les controles non-transgéniques issus de la même portée (littermate) (Fig2). Par contre, cette apoptose spontanée est fortement augmentée (6%) chez les souris transgéniques exprimant la mutation PS1-M146L ou la multimutation PS1M5.

De manière très intéressante, après culture pendant 2.5 h sans stimulus apoptotique, les niveaux d'apoptose spontanée pour les lymphocytes de PS1M5 sont plus élevés que pour PS1wt ou le simple mutant PS1M146L ou encore les controles non-transgéniques (Fig3).

Ces résultats démontrent que les cellules de l'animal transgénique de l'invention et en particulier celles des tissus périphériques (lymphocytes notamment) présentent non seulement une apoptose spontanée plus élévée par rapport aux contrôles mais également qu'elle persiste dans le temps.

### Exemple 3 : Augmentation de l'apoptose induite par traitement au déoxyribose dans les lymphocytes issus de souris transgéniques PS1M5.

Cet exemple a pour but de démontrer que l'apoptose induite par le déoxyribose est beaucoup plus forte dans le modèle de souris PS 1 multi-mutant.

Après induction par traitement au 2-deoxy-D-ribose (d-Rib), les niveaux d'apoptose sont significativement plus élevés pour les lymphocytes transgéniques PS1M5 que pour PS1wt , simple mutant PS1-M146L ou les controles non-transgéniques (Fig4).

Cette augmentation de l'apoptose après induction démontre une réponse accrue (sensibilisation) à un stress apoptotique due à l'expression de PS1 multimutante. Combiner avec une augmentation d'apoptose basale, ces résultats indiquent clairement que l'expression transgénique d'une forme multimutéede PS1 entraine une bien plus grande sensibilité à l'apoptose dans les lymphocytes.......

### Exemple 4 : Augmentation de l'apoptose induite par traitement au peroxyde d'hydrogène dans les lymphocytes issus de souris transgéniques PS1M5.

Cet exemple a pour but de démontrer que l'apoptose induite par le peroxyde d'hydrogène est beaucoup plus forte dans le modèle de souris PS1 multi-mutant.

Après stimulation par traitement au peroxide d'hydrogène (H2O2), les niveaux d'apoptose sont significativement plus élevés dans les lymphocytes PS1M5 que pour les autres transgéniques ou non-transgéniques (p< 0.001, Fig5). Il n'y a pas de différence significative entre les lymphocytes transgéniques PS1wt (S182) et simple mutant PS1M146L ou les controles non transgéniques.

Cette augmentation de l'apoptose après induction par un stress différent démontre la généralité de l'hypersensibilité à l'apoptose observée dans ce modèle.

### Exemple 5 : Augmentation de l'apoptose induite par traitement à la déxaméthasone dans les lymphocytes issus de souris transgéniques PS1M5.

Cet exemple a pour but de démontrer que l'apoptose induite par un traitement à la dexaméthasone est beaucoup plus forte dans le modèle de souris PS 1 multi-mutant.

De même, après stimulation par la dexaméthasone, les niveaux d'apoptose sont significativement plus élevés dans les lymphocytes PS1M5 que pour les autres transgéniques ou non-transgéniques (p< 0.01, Fig6). Il n'y a pas de différence significative entre les lymphocytes transgéniques PS1wt et simple mutant PS1M146L ou les controles non transgéniques.

Cette augmentation de l'apoptose après induction par un troisième stress différent confirme la généralité de l'hypersensibilité à l'apoptose conférée par l'expression transgénique de PS1 multimutée (mais pas simple mutant).

### Exemple 6 : Augmentation des niveaux de radicaux libres dans les lymphocytes de souris transgéniques PS1M5.

Les niveaux de agents radicalaires oxygénés (Reactive Oxygen Species) ont été mesurés dans les lymphocytes de souris par cytométrie de flux . Les taux de ROS sont significativement plus élevés dans les lymphocytes de souris transgéniques PS1M5 (p<0.05) par rapport aux autres groupes (Fig7). Il n'y a pas de différence significative entre les autres groupes bien qu'une tendance à l'augmentation des niveaux de ROS se dessine entre les controles littermates et les transgéniques PS1wt et PS1M146L.

Cette augmentation des niveaux de ROS démontre qu'on peut détecter en conditions basales dans ce modèle une altération d'un paramètre biochimique (fortement affecté dans l'AD) qui pourrait soutendre l'hypersensibilité à l'apoptose.

### Exemple 7 : Mobilisation accrue de calcium intracellulaire dans les lymphocytes de souris transgéniques PS1M5.

Parce que l'apoptose est modulée par les niveaux intracellulaires de calcium, ces derniers ont été analysés dans les lymphocytes des souris transgéniques. Au repos, les niveaux de [Ca²⁺]ᵢ pour les controles et les souris transgéniques PS1wt et PS1M146L sont identiques (inférieurs ou égaux à environ 150 nM). Le multiple mutant PS1M5 démontre une légère élévation des taux de base (supérieur à 200 nM) (Fig 8).

Après stimulation mitogénique (PHA, 15 microg/ml), les niveaux de calcium intracellulaire augmentent d'approximativement 70nM dans les souris contrôles et transgéniques PS1 wt et PS1M146L (Fig 9). Par contre, pour les souris PS1M5, cette augmentation est de 190 nM ce qui est statistiquement différent des autres groupes (p< 0.01). Donc, non seulement les niveaux de [Ca²⁺]ᵢ sont plus élevés en conditions basales, mais la réponse nette à un stimulus est elle-aussi plus importante résultant en des niveaux absolus beaucoup plus élevés (400 nM par rapport à 200-220 nM pour les autres groupes, différence statistiquement significative).

De plus par rapport aux controles non transgéniques qui exhibent une response calcique très rapide, la latence pour atteindre le pic de [Ca²⁺]ᵢ après stimulation au PHA est fortement augmentée dans les lymphocytes des transgéniques PS1M5 et à un moindre degré pour les PS1M146L (Fig 10) mais pas dans les PS1wt.

Les différences des niveaux et cinétiques des réponses calciques dans les lymphocytes démontrent une forte altération des processus de mobilisation des réserves intracellulaires de calcium due à l'expression de PS1 multimutant dans ce modèle. L'altération de la cinétique des réponses calciques avait été démontrée précédemment dans les lymphocytes des patients AD ce qui renforce la pertinence de ce modèle animal.

### Exemple 8 : Etude du métabolisme des radicaux libres dans le cerveau.

Pour confirmer la pertinence par rapport à la maladie d'Alzheimer des déficits observés chez les souris transgéniques de l'invention, il a été recherché si des altérations pathologiques étaient identifiables au niveau du cerveau de ces animaux. En particulier les mécanismes de protection contre les radicaux libres ont été analysés puisque ces derniers sont impliqués dans les phénomènes d'apoptose liés aux présénilines et qu' une altération dans les lymphocytes de souris transgéniques PS1M5 a été démontrée dans l'exemple 6.

Les anlayses ont été effectuées sur des animaux de 3-4 mois d'age. Par rapport aux souris transgéniques PS1wt, les PS1M146L démontrent une baisse des niveaux d'activité SOD d'approximativement 20% dans le cerveau (Fig11A). Cette diminution est encore accentuée chez les transgéniques PS1M5 (-28%, p< 0.05).

L'activité glutathion réductase est elle aussi significativement diminuée dans le cerveau des transgéniques PS1M5 (-27%, p< 0.05). A l'inverse, on note une diminution vraiment modeste de cette activité effet modeste chez les transgéniques

PS1-M146L (Fig11B).

Les niveaux de peroxydation lipidique en basal étaient identiques dans tous les groupes de souris. Après stimulation au FeCl₃, par contre, les niveaux de peroxydation lipidique sont accrus dans les transgéniques PS1M5 (+ 20%, Fig 11C).

Chez des jeunes adultes (3-4 mois) transgéniques PS1M5 multimutée, on observe donc un déficit des mécanismes de protection des radicaux libres et parallèlement une augmentation de la sensibilité à la peroxydation lipidique dans le cerveau. Cet effet correlle parfaitement avec la sensibilité accrue à l'apoptose, les altérations de mobilisation de calcium intracellulaire et l'augmentation des taux d'espèces radicalaires oxygénées observées dans les lymphocytes de ces transgéniques qui expriment artificiellement le transgène dans ces deux tissus. Le déficit des mécanismes de protection contre les radicaux libres a aussi été relevé chez les patients atteints de la maladie d'Alzheimer, confirmant ainsi la pertinence de ce modèle animal.

### Références :

Chui, D-H, Tanahashi, H., Ozawa, K., Ikeda, S., Checler, F., Ueda, O., Suzuki, H., Araki, W., Inoue, H., Shirotani, K., Takahashi, K., Gallyas, F. and Tabira, T. (1999) Transgenic mice withAlzheimer presenilin1 mutations show accelerated neurodegeneration without amyloid plaque formation. Nature Medecine 5 :560-564.
Czech, C., Delaere, P., Macq, A.-F., Reibaud, M., Dreisler, S., Touchet, N., Schomber, B., Mazadier, M., Mercken, L., Theisen, M., Pradier, L., Octave, J.-N., Beyreuther, K. and Tremp, G. L. (1997) Proteolytical processing of mutated human amyloid protein precursor in transgenic mice. Mol. Brain Res. 47, 108-116.
Czech, C., Lesort, M., Tremp, G., Terro, F., Blanchard, V., Schombert, B., Carpentier, N., Dreisler, S., Bonici, B., Takashima, A., Moussaoui, S., Hugon, J. and Pradier, L. (1998) Characterization of human presenilin 1 transgenic rats: increased sensitivity to apoptosis in primary neuronal cultures. Neuroscience 87, 325-36.
Eckert, A., Förstl H., Zerfass, R., Hennerici, M. and Müller, WE. (1997) Free intracellular calcium in peripheral cells in Alzheimer's Disease. Neurobiol. Aging 18 : 281-284.
Eckert, A., Förstl H., Zerfass, R., Oster, M., Hennerici, M. and Müller, WE. (1998) Changes in intracellular calcium regulation in Alzheimer's Disease and vascular dementia. J. Neural Transm. (Suppl.) 53 :259-267
Gautier, C., Methali, M. and Lathe, R. (1989) A ubiquitous mammalian expression vektor, pHMG, based on a housekeeping gene promoter. Nucleic. Acids. Res. 17, 8398
Luthi, A., Putten, H., Botteri, F. M., Mansuy, I. M., Meins, M., Frey, U., Sansig, G., Portet, C., Schmutz, M., Schroder, M., Nitsch, C., Laurent, J. P. and Monard, D. (1997) Endogenous serine protease inhibitor modulates epileptic activity and hippocampal long-term potentiation. J Neurosci 17, 4688-99.
Pradier, L., Carpentier, N., Delalonde, L., Clavel, N., Bock, M.-D., Buée1, L., Mercken, L., Tocqué, B. and Czech, C. (1999) Mapping the APP/presenilin (PS) binding domains: the hydrophilic N-terminus of PS2 is sufficient for interaction with APP and can displace APP/PS1 interaction. NEurobiol. Dis. 6, 43-55.
   Price DL and Sisodia SS (1998). Mutant genes in familial Alzheimer's disease and transgenic models. Annu. Rev. Neurosci. 21 :479-505.
Sasahara, M., Fries, J. W., Raines, E. W., Gown, A. M., Westrum, L. E., Frosch, M. P., Bonthron, D. T., Ross, R. and Collins, T. (1991) PDGF B-chain in neurons of the central nervous system, posterior pituitary, and in a transgenic model. Cell 64, 217-27.
Scott, M. R., Kohler, R., Foster, D. and Prusiner, S. B. (1992) Chimeric prion protein expression in cultured cells and transgenic mice. Protein Sci 1, 986-97.
Sherrington, R., Rogaev, E. L, Liang, Y., Rogaeva, E. A., Levesque, G., Ikeda, M., Chi, H., Lin, C., Li, G., Holman, K. et al. (1995) Cloning of a gene bearing missense mutations in early-onset familial Alzheimer's disease. Nature 375, 754-60.
Yankner BA (1996). Mechanisms of neuronal degeneration in Alzheimer's disease. Neuron.16 :921-932.

## Revendications

1. Animal transgénique non-humain exprimant une forme de la préséniline 1 portant au moins cinq mutations parmi les mutations de la préséniline 1 identifiées dans les formes familiales de la maladie d'alzheimer et présentant une sensibilité accrue à l'apoptose.

2. Animal selon la revendication 1, **caractérisé en ce que** les mutations dans le gène de la PS1 sont les mutations M146L, H163R, A246E, L286V, C410Y, 1143T, L235P, P264L, P267S, E317G, G384A, L392V, A426P et /ou P436S.

3. Animal selon l'une des revendications 1 et 2, **caractérisé en ce que** les mutations dans le gène de la PS1 sont les mutations M146L, H163R, A246E, L286V et C410Y combinées entre elles.

4. Animal selon l'une des revendications 1 à 3, **caractérisé en ce que** l'expression de la PS1 est placée sous le contrôle d'un promotteur ubiquitaire.

5. Utilisation d'un animal selon l'une des revendications 1 à 4 pour la mise en évidence de composés destinés au traitement des maladies neurodégénératives.

6. Utilisation d'un animal selon l'une des revendications 1 à 4 pour la mise en évidence de composés destinés au traitement de la maladie d'Alzheimer.

7. cellule extraite d'un animal selon l'une des revendications 1 à 4, la-dite cellule exprimant une forme de la préséniline 1 portant au moins cinq mutations parmi les mutations de la préséniline 1 identifiées dans les formes familiales de la maladie d'alzheimer et présentant une sensibilité accrue a l'apoptose.

8. Utilisation d'une cellule selon la revendication 7, pour la mise en évidence de composés destinés au traitement des maladies neurodégénératives.

9. Utilisation d'une cellule selon la revendication 7, pour la mise en évidence de composés destinés au traitement de la maladie d'Alzheimer.

## Claims

1. Nonhuman, transgenic animal expressing a form of presenilin 1 carrying at least five mutations among the presenilin 1 mutations identified in the familial forms of Alzheimer's disease and exhibiting increased sensitivity to apoptosis.

2. Animal according to Claim 1, **characterized in that** the mutations in the PS1 gene are the mutations the mutations M146L, H163R, A246E, L286V, C410Y, I143T, L235P, P264L, P267S, E317G, G384A, L392V, A426P and/or P436S.

3. Animal according to either of Claims 1 and 2, **characterized in that** the mutations in the PS1 gene are M146L, H163R, A246E, L286V and C410Y combined with each other.

4. Animal according to one of Claims 1 to 3, **characterized in that** the expression of PS1 is placed under the control of ubiquitous promoter.

5. Use of an animal according to one of Claims 1 to 4, for the detection of compounds intended for the treatment of neurodegenerative diseases.

6. Use of an animal according to one of Claims 1 to 4 for the detection of compounds intended for the treatment of Alzheimer's disease.

7. Cell extracted from an animal according to one of Claims 1 to 4, the said cell expressing a form of presenilin 1 carrying at least five mutations among the presenilin 1 mutations identified in the familial forms of Alzheimer's disease and exhibiting increased sensitivity to apoptosis.

8. Use of a cell according to Claim 7, for the detection of compounds intended for the treatment of neurodegenerative diseases.

9. Use of a cell according to Claim 7, for the detection of compounds intended for the treatment of Alzheimer's disease.

## Patentansprüche

1. Nichtmenschliches transgenes Tier, das eine Form von Presenilin 1 exprimiert, die mindestens fünf Mutationen, ausgewählt aus den Presenilin-1-Mutationen, die in den familiären Formen von Morbus Alzheimer auftreten und die eine erhöhte Sensitivität gegenüber Apoptosis aufweisen, trägt.

2. Tier nach Anspruch 1, **dadurch gekennzeichnet, dass** es sich bei den Mutationen in dem PS1-Gen um die Mutationen M146L, H163R, A246E, L286V, C410Y, I143T, L235P, P264L, P267S, E317G, G384A, L392V, A426P und/oder P436S handelt.

3. Tier nach einem der Ansprüche 1 und 2, **dadurch gekennzeichnet, dass** es sich bei den Mutationen in dem PS1-Gen um die Mutationen M146L, H163R, A246E L286V und C410Y handelt, die miteinander kombiniert sind.

4. Tier nach einem der Ansprüche 1 bis 3, **dadurch gekennzeichnet, dass** die Expression von PS1 unter die Kontrolle eines ubiquitären Promoters gestellt wird.

5. Verwendung eines Tiers nach einem der Ansprüche 1 bis 4 zum Nachweisen von Verbindungen für die Behandlung von neurodegenerativen Krankheiten.

6. Verwendung eines Tiers nach einem der Ansprüche 1 bis 4 zum Nachweisen von Verbindungen für die Behandlung von Morbus Alzheimer.

7. Zellextrakt eines Tiers nach einem der Ansprüche 1 bis 4, wobei die Zelle eine Form von Presenilin 1 exprimiert, die mindestens fünf Mutationen, ausgewählt aus den Presenilin-1-Mutationen, die in den familiären Formen von Morbus Alzheimer auftreten und die eine erhöhte Sensitivität gegenüber Apoptosis aufweisen, trägt.

8. Verwendung einer Zelle nach Anspruch 7 zum Nachweisen von Verbindungen zur Behandlung von neurodegenerativen Krankheiten.

9. Verwendung einer Zelle nach Anspruch 7 zum Nachweisen von Verbindungen für die Behandlung von Morbus Alzheimer.
